**Europäisches Patentamt**

**European Patent Office**

**Office européen des brevets**

(19)

(11) Publication number: **0 080 330**
**B1**

(12) # EUROPEAN PATENT SPECIFICATION

(45) Date of publication of patent specification: **24.09.86**

(21) Application number: **82306146.0**

(22) Date of filing: **18.11.82**

(51) Int. Cl.⁴: **A 01 N 25/12,** A 01 N 25/26,
A 01 N 25/34, A 61 K 9/22,
A 61 K 9/26, A 61 K 9/52,
A 61 K 9/70, C 05 G 3/00

(54) **Composite materials.**

(30) Priority: **18.11.81 GB 8134752**
**05.11.82 GB 8231654**

(43) Date of publication of application:
**01.06.83 Bulletin 83/22**

(45) Publication of the grant of the patent: .
**24.09.86 Bulletin 86/39**

(84) Designated Contracting States:
**BE CH DE FR IT LI NL SE**

(56) References cited:
**EP-A-0 024 891**
**FR-A-2 297 195**
**GB-A-1 425 550**
**GB-A-1 503 116**
**GB-A-1 512 637**
**GB-A-1 565 906**
**GB-A-1 604 630**
**US-A-4 015 970**
**US-A-4 098 610**
**US-A-4 237 114**
**US-A-4 299 613**

(73) Proprietor: **STC plc**
**190 Strand**
**London, WC2R 1DU (GB)**

(72) Inventor: **Drake, Cyril Francis**
**1 Church Cottages**
**Harlow Essex (GB)**
Inventor: **Jones, Ronald**
**14 Leat Close**
**Sawbridgeworth Hertfordshire (GB)**

(74) Representative: **Dennis, Mark Charles et al**
**STC Patent Department Edinburgh Way**
**Harlow Essex CM20 2SH (GB)**

Courier Press, Leamington Spa, England.

## Description

This invention relates to arrangements and methods for the controlled release of active materials into solution.

There is a broad range of applications wherein it is necessary to provide for the release of an active material at a controlled rate into an aqueous environment. In the biosciences, particularly, the potential for prolonging the action of numerous bioactive compounds is stimulating considerable interest.

Since the early 1950's researchers have attempted to develop controlled release compounds able to store active materials and then release them at controlled rates into aqueous systems. This research has tended to concentrate on polymeric materials. Many polymers may be fabricated at relatively low temperatures to encapsulate effectively active materials thereby protecting them from unwanted interaction with the environment. Subsequent release of the active material is effected by one of four general mechanisms, i.e. diffusion, swelling, (bio) chemical action and magnetic processes. However, in most cases it has proved impractical to engineer the required degree of control, especially over long periods, and in many instances toxicity of the special polymer itself has limited exploitation.

Various techniques for releasing organic materials have been proposed. For example, U.S. patent specifications 4,166,111 and 4,299,613 refer to a polymer body containing (a) one or more active materials and (b) a dispersed water soluble powder whereby leaching of the matrix to release the active material is enhanced. The volume proportion of the powder in the matrix is significantly less than 50%. The dissolution of the active material from such a device is limited by diffusion and therefore decays exponentially with time.

Controlled Release Glasses (CRG), such as those described in our co-pending application No. EP—A—0 024 891, are inorganic glasses that may be formulated to be non-toxic. Their solution rates in aqueous media are chemically controlled and may be varied to give a high degree of precision, even over long periods. Unfortunately CRG may only be fabricated at temperatures that would, on contact, destroy or degrade many of the active materials of interest. For this reason the development of CRG has tended to be concentrated on applications requiring the release of inorganic materials.

The object of this invention is to combine the most advantageous properties of both the polymer and CRG approaches, without risk of toxicity. Thus the benefits of low temperature fabrication and release control combine to provide a new means of releasing a wide range of active materials.

According to the invention there is provided a composite body for the release of an active material into an aqueous medium by dissolution of the material from a polymer host, characterised in that the body comprises a polymer in which the active material is dispersed and a particulate water soluble glass, that, in use, exposure of the polymer to the aqueous medium is effected by dissolution of the glass to provide passageways into the polymer, that the dissolution rate of the glass, and thus the rate at which the polymer is exposed to effect release of the active material, is predetermined by control of the glass composition, and that the particulate glass comprises at least 50% by volume of the composite material whereby substantially each glass particle is in contact with adjacent glass particles.

When the composite comes into contact with an aqueous medium the particulate material dissolves to provide a series of passageways through the host matrix thus permitting the ingress of water. Such a composite can be employed for the delayed and controlled release of an active material and the consequent significant increase in the water permeability of the composite, is protected by the composite itself.

The particulate glasses may comprise a single glass composition or a mixture of glass compositions. Water soluble glass compositions have the important property that their dissolution rate may be tailored to a desired value by composition adjustment thus providing for the manufacture of composite materials with a wide range of release characteristics. Moreover, many of these glasses are biologically inert.

Embodiments of the invention will now be described with reference to the accompanying drawings in which:—

Fig. 1 is a cross section of a portion of a composite structure; and

Fig. 2 illustrates the effect of an aqueous medium on the composite structure shown in Fig. 1.

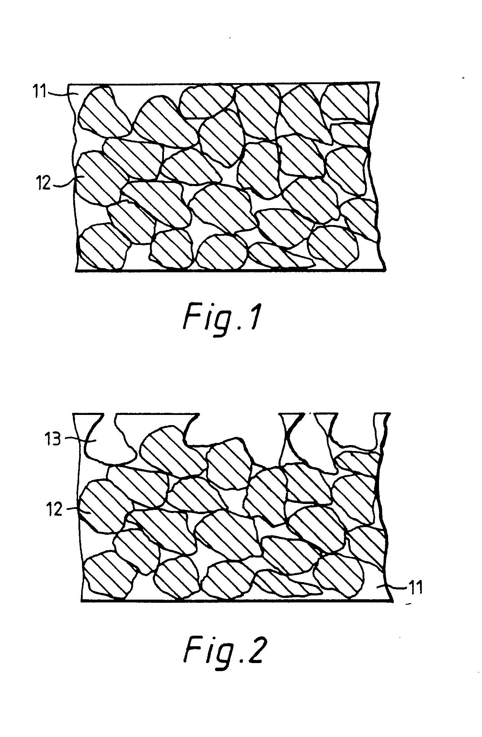

Referring to the drawings, the composite shown comprises a host matrix 11, of a relatively low solubility or of essentially insoluble polymer, in which matrix a soluble particulate water soluble glass 12 is dispersed. The loading of the particulate glass 12 in the polymer 11 is sufficient to ensure that substantially each particle 12 is in contact with at least two similar particles. When the structure is placed in water or an aqueous medium the particulate glass 12 dissolves to provide an array of passageways 13 (Fig. 2) through the polymeric matrix thus significantly increasing the water permeability of the composite.

The composite may be employed to provide for the controlled release of an active material that is either dispersed within it or is coated with it or covered by it.

The polymeric matrix 11 may comprise a wide variety of materials. For biological and medical applications we prefer to employ cellulosic materials, but other materials may of course also be employed. Typical of such polymers, though by no means limiting, are polyene, polyesters, polyamides, polysaccharides, natural gums and latexes, and mixtures and copolymers thereof. Advantageously, in some applications, the polymer matrix may be thermosetting or thermoplastic thus permitting low cost bulk fabrication by any of the known techniques, e.g. pressing or extruding. Equally the polymer may be crosslinked by any of the known techniques.

The dissolution rate in an aqueous medium of the water soluble glass 12 is determined by the relative proportions of the various glass forming and glass modifying constituents.

We prefer to employ phosphate or borate glasses for this purpose as such materials are relatively non-toxic and also simple to prepare. Furthermore, the techniques for controlling the dissolution rate of these glass systems are well understood. The glasses generally comprise phosphorus pentoxide, boric oxide or mixtures thereof, as the glass forming oxide of the glass together with one or more further oxides which provide the glass modifying constituents of the composition. The dissolution rate, and the pH of solutions of such compositions are determined by the nature and proportion of the glass modifying oxide or oxides and by the overall molar ratio of glass modifier to glass former.

We have also found that the presence of certain metal oxides, for example the oxides of most two or three valent metals, reduces the dissolution rate of a glass composition whilst the presence of other metal oxides, in particular alkali metal oxides, increases the dissolution rate. Thus, by suitable adjustment of the ratio of glass-formers to glass modifiers and proportions of these two types of oxides a wide range of dissolution rates can be obtained. The techniques of glass dissolution rate and solution pH control are more fully described in our copending application No. EPA 0024891.

Typical of suitable glass compositions, but by no means limiting, are alkali metal/phosphate glasses and alkali metal/borate glasses.

The composite material may be manufactured by a number of techniques according to the ultimate application of the material. Thus the particulate water soluble glass may be mixed with the solid or liquid polymer and cast, pressed or moulded to form monolithic blocks or the two may be extruded together into rods, tubes, or fibres. Other techniques include spray granulations of mixtures of the particulate material with the polymers.

In all these processes the volume proportion and/or form of distribution of the particulate water soluble glass in the composite structure should be sufficient to ensure that substantially each particle is in contact with at least two similar particles. This requires a volume proportion of at least 50% of the total volume and preferably at least 70%. In some applications the composite may contain as much as 90 volume % of the particulate water soluble glass, this high concentration being achieved by the incorporation of a mixture of large and small particles into the polymer.

The body of materials described herein may be used in a variety of applications as structures whereby an active material is stored and then subsequently released into an aqueous medium at a controlled and predetermined rate. In many applications it may be desirable to minimise the water permeability of the composite prior to dissolution of the glass. This requires that the glass be wetted by the polymer. Since the glasses described herein are typically hydrophylic we prefer, in such applications, to select polymers that are not non-polar.

Where the body is in the form of a monolithic block or granules containing one or more active materials either homogeneously or heterogeneously dispersed throughout it and is subsequently immersed in an aqueous medium, the particulate water soluble glass dissolves thus providing passageways through the polymer. This significantly increases the water permeability of the composite and allows the active material to dissolve out. Such an arrangement is suitable for bulk distribution of an active material which material may comprise e.g. a fertiliser, a selective or non-selective herbicide, an insecticide, a pheromone, a moluscicide, a nematicide, a fungicide, an algicide, a slimicide, a rodenticide, or mixtures thereof. The applications of such a structure include, but are not limited to, soil and crop treatment, biocidal purification of water courses, corrosion protection and the inhibition of bacterial growth in water systems. In a further application a medicament or drug may be incorporated in the structure which may be administered to a human or animal patient either internally, in the form of a tablet, implant or suppository (or a bolus for ruminant animals), or externally as a percutaneous device. The medicament or drug may comprise a nutrient, a prophylactic agent, a therapeutic agent, an antibiotic, an antiseptic agent, a parasiticide, a hormone or contraceptive.

The monolithic body may be formed into a single or multi-cavity structure the cavities of which contain a liquid or solid drug or medicament. A single cavity structure of this kind, when contacted by an aqueous medium, provides a controlled delay prior to release of the active content. Where a multicavity device is employed, a predetermined or programmed release rate profile may be obtained.

It is often advantageous to employ a polymeric material that has an appreciable but very low dissolution rate or that is biodegradable. This ensures that ultimately no solid residue is left.

Moreover, in the case where the active material is dispersed within the polymeric matrix the release rate profile is determined by the relative rate of dissolution of the particulate water soluble glass to the dissolution, degradation, or erosion rate of the polymer. It can be shown that if the former dissolution rate is significantly greater than the latter then the rate of transfer of the active material to solution follows approximately a square root law, whereas if the two rates are similar then the rate of transfer approximates to a linear law.

In a particularly advantageous arrangement the active material is granular and is formed into a body wherein the granules are cemented together by a composite comprising a polymer and a powdered water soluble glass. The glass particles are significantly smaller than the active material granules so that the composite acts as a 'mortar' between the granules thus providing a coherent body. The body may be formed e.g. by pressing, extrusion or casting.

The encapsulated body may comprise a pill or tablet for oral administration to a human or animal patient, the coating providing a predetermined delay prior to release of the active substances contained in the pill or tablet. In a particularly advantageous arrangement a plurality of such devices, each with a different dissolution rate or different thickness coating, are sealed in a single soluble, e.g. gelatin, casing. When this device is administered to a patient the casing dissolves releasing the individual capsules each of which then releases its active content after a different predetermined delay. This provides the patient with regular doses of the active material over a period of several hours or days.

In a further embodiment the active material is in granular form and the individual granules are each coated with a film to produce a granular product in which, after coming into contact with an aqueous medium, there is a delay before the active material starts to dissolve in the aqueous medium.

The composite material may be applied as a surface coating on a solid substrate from a slurry of the particulate material in a thermosetting polymer. The slurry is applied to the surface by brushing, spreading or spraying and is then cured to a coherent material by heating, chemical crosslinking, physical cross linking, solvent evaporation, or by applying energy in the form of light or other electromagnetic radiation. In such an arrangement an active material is incorporated in the composite, the active material being released when dissolution of the particle material allows water to diffuse into the composite. The active material for such applications may be an antifoulant, e.g. for marine use, a corrosion inhibiting agent or an antiseptic material.

## Claims

1. A composite body for the release of an active material into an aqueous medium by dissolution of the material from a polymer host, characterised in that the body comprises a polymer in which the active material is dispersed and a particulate water soluble glass, that, in use, exposure of the polymer to the aqueous medium is effected by dissolution of the glass to provide passageways into the polymer, that the dissolution rate of the glass, and thus the rate at which the polymer is exposed to effect release of the active material, is predetermined by control of the glass composition, and that the particulate glass comprises at least 50% by volume of the composite material whereby substantially each glass particle is in contact with adjacent glass particles.

2. A body as claimed in claim 1, characterised in that the particulate glass comprises a phosphate or borate glass.

3. A body as claimed in claim 1 or 2 in the form of a monolithic block.

4. A body as claimed in any one of claims 1 to 3, characterised in that the active material comprises a fertiliser, a selective or non-selective herbicide, an insecticide, a pheromone, a molluscide, a larvicide, a nematocide, a fungicide, an algicide, a slimicide, a rodenticide, or mixtures thereof.

5. A body as claimed in any one of claims 1 to 3, characterised in that the active material comprises a nutrient, a prophylactic agent, a therapeutic agent, an antibiotic, an antiseptic agent, a parasiticide, a hormone, or contraceptive.

6. A body as claimed in any one of claims 1 to 5, characterised in that the particulate glass comprises 50—90% by volume of the composite material.

7. A body as claimed in claim 6, characterised in that the glass comprises at least 70% by volume of the composite material.

8. A body as claimed in any one of claims 1 to 7, characterised in that the active material is incorporated in cavities in the body.

9. A body as claimed in any one of claims 1 to 8, characterised in that the particulate glass comprises a mixture of glass compositions.

10. A therapeutic device being an implant or percutaneous device, a bolus or other device capable of oral, rectal or surgical administration comprising a body as claimed in any one of claims 1 to 9.

11. A device as claimed in claim 10, characterised in that it comprises a surface coating on a solid carrier body.

12. A device as claimed in claim 10, characterised in that said active material is an antirejection agent, an adjuvant or an antiseptic.

## Patentansprüche

1. Zusammengesetzter Körper für das Freisetzen von Aktivmaterial in ein wässeriges Medium durch Auflösen des Materials aus einem polymeren Träger, dadurch gekennzeichnet, daß der Körper ein Polymer, in dem das Aktivmaterial verteilt ist, und ein aus einzelnen Teilchen bestehendes, wasserlösliches Glas enthält, daß das Polymer im Gebrauch dem wässerigen Medium ausgesetzt wird, indem das Glas aufgelöst wird und Durchgänge in dem Polymer freigegeben werden, daß die Auflösungsgeschwindigkeit des Glases und damit die Geschwindigkeit, mit der das Polymer zur Freisetzung von Aktivmaterial bloßgelegt wird, durch Steuerung der Glaszusammensetzung vorbestimmt wird und daß das aus einzelnen Teilchen bestehende Glas zumindest 50 Volumenprozent des zusammengesetzten Körpers aufweist, wobei im wesentlichen jedes Glasteilchen in Kontakt mit angrenzenden Glasteilchen steht.

2. Körper nach Anspruch 1, dadurch gekennzeichnet, daß das aus einzelnen Teilchen bestehende Glas Phosphat- oder Boratglas enthält.

3. Körper nach Anspruch 1 oder 2, in Form eines monolithischen Blockes.

4. Körper nach einem der Ansprüche 1 bis 3, dadurch gekennzeichnet, daß das Aktivmaterial ein Düngemittel, ein selektives oder nicht-selektives Herbizid, ein Insektizid, ein Pheromon, ein Molluskizid, ein Larvizid, Nematozid, ein Fungizid, ein Algizid, ein Slimizid (Antischleimmittel), ein Rodentizid oder Mischungen davon enthält.

5. Körper nach einem der Ansprüche 1 bis 3, dadurch gekennzeichnet, daß das Aktivmaterial ein Nährmittel, ein Prophylaksemittel, ein therapeutisches Mittel, ein antibiotisches Mittel, ein antiseptisches Mittel, ein Parasitizid, ein Hormon oder ein empfängnisverhütendes Mittel enthält.

6. Körper nach einem der Ansprüche 1 bis 5, dadurch gekennzeichnet, daß das aus einzelnen Teilchen bestehende Glas 50 bis 90 Volumenprozent des zusammengesetzten Materials enthält.

7. Körper nach Anspruch 6, dadurch gekennzeichnet, daß das Glas zumindest 70 Volumenprozent des zusammengesetzten Materials enthält.

8. Körper nach einem der Ansprüche 1 bis 7, dadurch gekennzeichnet, daß das Aktivmaterial in Hohlräume des Körpers eingebaut ist.

9. Körper nach einem der Ansprüche 1 bis 8, dadurch gekennzeichnet, daß das aus einzelnen Teilchen bestehende Glas eine Mischung von Glaszusammensetzungen enthält.

10. Therapeutisches Mittel oder Einrichtung als Einsatz oder zu perkutanen Anwendung, Bolus (Bissen) oder anderes Mittel geeignet zur oralen, rektalen oder chirurgischen Handhabung, enthaltend einen Körper nach einem der Ansprüche 1 bis 9.

11. Mittel nach Anspruch 10, dadurch gekennzeichnet, daß es eine Oberflächenbeschichtung auf einem festen Trägerkörper aufweist.

12. Mittel nach Anspruch 10, dadurch gekennzeichnet, daß das Aktivmaterial ein Antiausscheidungsmittel, ein Adjuvant oder ein antiseptisches Mittel ist.

**Revendications**

1. Corps composite pour libérer une matière active et la dégager dans un milieu aqueux par dissolution de la matière à partir d'un hôte polymère, corps caractérisé en ce qu'il comprend un polymère dans lequel la matière active est dispersée et un verre particulaire hydrosoluble; en ce que, en service, l'exposition du polymère au milieu aqueux est réalisée par dissolution du verre pour constituer des passages dans le polymère; en ce que la vitesse de dissolution du verre et, ainsi, la vitesse à laquelle le polymère est exposé pour effectuer une libération de la matière active, est prédéterminée par réglage de la composition du verre; et en ce que le verre particulaire constitue au moins 50 % du volume de la matière composite, de sorte que pratiquement chaque particule de verre est en contact avec des particules adjacentes de verre.

2. Corps selon la revendication 1, caractérisé en ce que le verre particulaire comprend un verre au phosphate ou au borate.

3. Corps selon la revendication 1 ou 2, sous forme d'un bloc monolithique.

4. Corps selon l'une quelconque des revendications 1 à 3, caractérisé en ce que la matière active comprend un engrais, un herbicide sélectif ou non sélectif, un insecticide, une phéromone, un molluscicide, un larvicide, un nématocide, un fongicide, un algicide, un destructeur de myxomycètes ou de générateurs de boues, un rodenticide ou un de leurs mélanges.

5. Corps selon l'une quelconque des revendications 1 à 3, caractérisé en ce que la matière active comprend une matière nutritive, un agent prophylactique, un agent thérapeutique, un antibiotique, un agent antiseptique, un parasiticide, une hormone ou un contraceptif.

6. Corps selon l'une quelconque des revendications 1 à 5, caractérisé en ce que le verre particulaire constitue 50 à 90 % du volume de la matière composite.

7. Corps selon la revendication 6, caractérisé en ce que le verre constitue au moins 70 % du volume de la matière composite.

8. Corps selon l'une quelconque des revendications 1 à 7, caractérisé en ce que la matière active est incorporée dans des cavités du corps.

9. Corps selon l'une quelconque des revendications 1 à 8, caractérisé en ce que le verre particulaire comprend un mélange de compositions de verre.

10. Dispositif thérapeutique qui est un implant ou un dispositif percutané, une grosse pilule ou un autre dispositif susceptible d'être administré par voie orale, rectale ou chirurgicale, comprenant un corps tel que revendiqué dans l'une quelconque des revendications 1 à 9.

11. Dispositif selon la revendication 10, caractérisé en ce qu'il comprend un revêtement de surface sur un corps de support solide.

12. Dispositif selon la revendication 10, caractérisé en ce que ladite matière active est un agent anti-rejet, un adjuvant ou un antiseptique.

Fig.1

Fig.2